# EUROPEAN PATENT APPLICATION

(11) **EP 4 491 179 A1**
(43) Date of publication of application: **15.01.2025**
(21) Application number: 24769658.6
(22) Date of filing: 22.01.2024
(51) Int. Cl.: A61K 31/433, A61K 38/12, A61P 39/02, A61P 13/12, A61P 31/04

(54) **USE OF SU3327 IN PREPARATION OF DRUG FOR REDUCING CYTOTOXICITY AND RENAL TOXICITY OF POLYMYXIN**

(30) Priority: 10.03.2023 CN 202310225879
(71) Applicant: Xiamen Hanlixin Pharmaceutical Co., Ltd., Xiamen, Fujian 361000 (CN); China Agricultural University, Haidian District, Beijing 100000 (CN)
(72) Inventor: DAI, Chongshan, Beijing 100000 (CN); CHEN, Hongliang, Xiamen, Fujian 361000 (CN); WU, Congming, Beijing 100000 (CN); ZHENG, Hailing, Xiamen, Fujian 361000 (CN); SHEN, Jianzhong, Beijing 100000 (CN)
(74) Representative: ABG Intellectual Property Law, S.L.
(86) International application number: PCT/CN2024/073408
(87) International publication number: WO 2024/187962

(57) **Abstract**

Use of a C-JUN N-terminal kinase inhibitor, SU3327, in the preparation of a medicament for reducing cytotoxicity and nephrotoxicity of polymyxin, wherein the polymyxin is preferably Polymyxin E. A treatment with a combination of SU3327 and Polymyxin E can significantly inhibit cytotoxicity and nephrotoxicity of Polymyxin E.

## Description

### Technical Field

The present invention relates to the field of medicine, and in particular, to the antibacterial field, particularly to a method for reducing cytotoxicity and nephrotoxicity of polymyxin by using SU3327.

### Background

SU3327 (also known as halicin, or Hailixin, CAS No.: 40045-50-9) is an N-terminal kinase inhibitor of C-JUN. SU3327 is an effective, selective, and substrate-competitive JNK inhibitor with an IC₅₀ of 0.7µM. SU3327 also inhibits protein interaction between JNK and JIP with an IC₅₀ value of 239nM. SU3327 has low activity for p38α and Akt kinases.

A research group led by James Collins, a biologist on synthetic biology, at the Massachusetts Institute of Technology, reported in its paper on an innovative machine learning approach for research and development, *"*A Deep Learning Approach to Antibiotic Discovery" that they had successfully discovered a novel antibiotic - halicin through experiments (the results of the study were published in Cell on February 20, 2020). James Collins's group combined the intelligent "antimicrobial neural network screening" with "toxicity screening", followed by deletion of compounds considered too much similar to the existing antibiotics (as bacteria might have developed resistance thereto). Finally, they considered as the most potential drug candidate a c-Jun N-terminal kinase inhibitor, SU3327 (which may be useful in the treatment of diabetes), a compound named "halicin" by the researchers. Laboratory tests showed that halicin not only effectively prevented the growth of *E. coli,* but also killed other bacteria, including *Mycobacterium tuberculosis, Clostridium difficile,* and many other drug-resistant bacteria that cause septicemia, pneumonia, wound infections, and other common refractory infections.

Polymyxin, an antibacterial polypeptide found in the culture solution of *Bacillus polymyxa,* is classified into five types, i.e., A, B, C, D and E. The antibacterial spectrum is similar with each other and has a broad range, and is found to have a strong effect, especially, on gram-negative bacteria, such as *E. coli, Bacillus pneumonia,* and *Pseudomonas aeruginosa.* Polymyxin E (also known as Polymyxin E, or colistin), CAS No.: 1066-17-7, English Name: Colistin, Trade Names: Kangdisu, Kelisiding and Nianjunsu. They are mainly used in sulfates or methanesulfonates for clinical application, i.e., Polymyxin E sulfate, and Polymyxin E methanesulfonate.

The side effects of polymyxin are mainly manifested in (1) nephrotoxicity, a common and serious side effect that may cause the regression of renal function in patients, with possible proteinuria, cylinderuria, azotemia, and elevated levels of creatinine - main damages to renal function; (2) certain neurotoxicity, polymyxin may cause dizziness, ataxia, lethargy and peripheral paresthesia in patients when used, all of which are side effects caused by polymyxin in nervous system. Nephrotoxicity and neurotoxicity are the most common toxic and side effects from Polymyxin E in clinical application, with a clinical incidence for nephrotoxicity accounting for up to 60%, neurotoxicity about 7%, and more significant symptoms from mild peripheral neurotoxicity, even as high as 100%. In addition, polymyxin still has such clinical application problems as "narrow" therapeutic window, transmitted drug resistance etc.

In order to enhance the therapeutic efficacy and reduce the cytotoxic effect of polymyxin, there remains a great demand in the art for agents provided with reduced cytotoxicity and nephrotoxicity of polymyxin. To date, there is no study reporting use of SU3327 in reducing cytotoxicity and nephrotoxicity of polymyxin.

### Summary

In order to solve the technical problem, i.e., cytotoxicity generated during treatment with polymyxin, the present invention provides a method for reducing cytotoxicity and nephrotoxicity of polymyxin, by using in particular a C-JUN N-terminal kinase inhibitor, SU3327, in combination with polymyxin, preferably Polymyxin E. Use of them in combination could not only achieve synergistic, enhanced antibacterial effect, but also increase cell viability to some extent upon treatment of polymyxin in combination with SU3327, compared with the treatment group with polymyxin alone. With certain, preferred concentration of SU3327, cell viability was increased to 68.5% due to the combination use of the two, showing an extremely significant difference compared with a control. It is suggested that combined treatment with SU3327 and Polymyxin E could significantly inhibit cytotoxicity of Polymyxin E. Further, in an established model of mice having kidney damage induced by Polymyxin E, treatment with Polymyxin E significantly reduced the kidney function of the mice, which was specifically exhibited as significantly elevated levels of urea nitrogen and creatinine in the serum, and at the same time resulted in obvious tubular necrosis, epithelial cell shedding, and the emergence of tubular casts. Following treatment in combination with SU3327, the levels of urea nitrogen and creatinine in the serum were significantly lowered, and the pathological changes in renal tubules were remarkably improved, suggesting that treatment with SU3327 in combination with Polymyxin E could significantly reduce nephrotoxicity caused by Polymyxin E.

One aspect of the present invention provides a use of a C-JUN N-terminal kinase inhibitor, SU3327, in the preparation of a medicament for reducing cytotoxicity and/or nephrotoxicity of polymyxin.

Another aspect of the present invention provides a use of a composition of a C-JUN N-terminal kinase inhibitor, SU3327, and polymyxin in the preparation of a medicament for reducing cytotoxicity and/or nephrotoxicity of polymyxin.

Further, the polymyxin is Polymyxin E (i.e., colistin) or Polymyxin B.

Further, the C-JUN N-terminal kinase inhibitor, SU3327, and Polymyxin E can be prepared into a composition for reducing cytotoxicity or nephrotoxicity in human or animal bodies caused by Polymyxin E.

Further, a mass ratio of SU3327 to polymyxin in the composition of SU3327 and polymyxin is: (2.5-10): 1.

Further, the dosage form of the composition of SU3327 and polymyxin is one of tablets, capsules, sustained release tablets, controlled release tablets, oral solutions, syrups, dosage forms for injection, dripping pills, and dosage forms of lyophilized powders for injection.

Further, the final, used concentration of Polymyxin E is 2 mM, and the final, used concentration of SU3327 is 5µM-0.625µM when used to reduce cytotoxicity; preferably, the final, used concentration of SU3327 is 2.5µM.

Further, treatment with SU3327 in combination significantly reduces nephrotoxicity induced by Polymyxin E, and the final, used dose concentration of SU3327 is 2.5mg/kg body weight, 5mg/kg body weight, and 10mg/kg body weight per day, and the used dose of Polymyxin E is 20mg/kg body weight per day.

The new use of a C-JUN N-terminal kinase inhibitor, SU3327, provided by the present invention - a method for reducing cytotoxicity of polymyxin - has excellent technical effects as follows:
(1) Use of SU3327 in combination with polymyxin could not only achieve synergistic, enhanced antibacterial effect, but also increase cell viability to some extent upon treatment of SU3327 in combination with polymyxin, compared with the treatment group with polymyxin alone. When SU3327 is used in a preferred, final concentration of 2.5µM, cell viability was increased to 68.5% due to the combination use of the two, showing an extremely significant difference compared with a control. It is suggested that combined treatment with SU3327 and Polymyxin E could significantly inhibit cytotoxicity of Polymyxin E.
(2) In an established model of mice having kidney damage induced by Polymyxin E, treatment with Polymyxin E significantly reduced the kidney function of the mice, which was specifically exhibited as significantly elevated levels of urea nitrogen and creatinine in the serum, and at the same time resulted in obvious tubular necrosis, epithelial cell shedding, and the emergence of tubular casts. Following treatment in combination with SU3327, the levels of urea nitrogen and creatinine in the serum were significantly lowered, and the pathological changes in renal tubules were remarkably improved, suggesting that treatment with SU3327 in combination with Polymyxin E could significantly reduce nephrotoxicity caused by Polymyxin E.
(3) The present invention provides an approach for addressing the problem of significant toxic and side effects of polymyxin in clinical application, which can solve the technical problems such as low clinical therapeutic index of polymyxin etc.

### Brief Description of Drawings

Fig. 1 A graph showing the results of reducing cytotoxicity of Polymyxin E to HEK293 cells following treatment with SU3327, including the results of cell viability tests following treatment with SU3327 alone, Polymyxin E alone, or in combination, according to the invention.
Fig. 2 A graph showing the detection results of urea nitrogen in the serum of the mice in control group, Polymyxin E model group, SU3327 control group, SU3327 high-dose protection group, SU3327 medium-dose protection group, and SU3327 low-dose protection group, according to the invention.
Fig. 3 A graph showing the detection results of creatinine in the serum of the mice in control group, Polymyxin E model group, SU3327 control group, SU3327 high-dose protection group, SU3327 medium-dose protection group, and SU3327 low-dose protection group, according to the invention.
Fig. 4 An image showing the histopathological sections of the mice in control group, Polymyxin E model group, SU3327 control group, SU3327 high-dose protection group, SU3327 medium-dose protection group, and SU3327 low-dose protection group, according to the invention.
Fig. 5 A graph showing the semi quantitative evaluations of control group, Polymyxin E model group, SU3327 control group, SU3327 high-dose protection group, SU3327 medium-dose protection group, and SU3327 low-dose protection group, according to the invention.

### Detailed Description

Medicaments used in the examples of the present invention: SU3327, purchased from MedChemexpress CO., Ltd, USA, purity ≥ 99%; Polymyxin E sulfate, purchased from Hebei Sacred Snow Dacheng Tangshan Pharmaceutical Co., Ltd., titer ≥ 23,000U/mg Polymyxin E. A certain amount of Polymyxin E sulfate was weighed to formulate an aqueous solution with a stock solution concentration of 16mg/mL. SU3327 was formulated with DMSO into a stock solution with a stock solution concentration of 40mg/mL. All stock solutions were prepared and kept in a -20°C refrigerator.

### Example 1 Protection by SU3327 against Cytotoxicity of Polymyxin

### 1.1 Cell Line Tested

Human Embryonic Kidney cells (HEK293), preserved by National Center for Veterinary Drug Safety Evaluation of China Agricultural University.

### 1.2 Cell Viability Test

Cytotoxicity was determined by a CCK-8 method. After HEK293 cells (5×10⁴ cells/well) were seeded onto 96-well tissue culture plates and cultured for 24 hours, the cells were treated with SU3327 or Polymyxin E alone or in combination, with SU3327 in a final concentration of 5µM, 2.5µM, 1.25µM and 0.625µM; Polymyxin E in a final concentration of 2mM; combination groups of SU3327 and Polymyxin E: Polymyxin E (2mM) + SU3327 (5µM), Polymyxin E (2mM) + SU3327 (2.5µM), Polymyxin E (2mM) + SU3327 (1.25µM), Polymyxin E (2mM) + SU3327 (0.625µM), and control group (Control) 0.1% DMSO. Following continued culture for 24h, a CCK-8 kit was used to detect the changes in cell viability.

### 1.3 Test Results

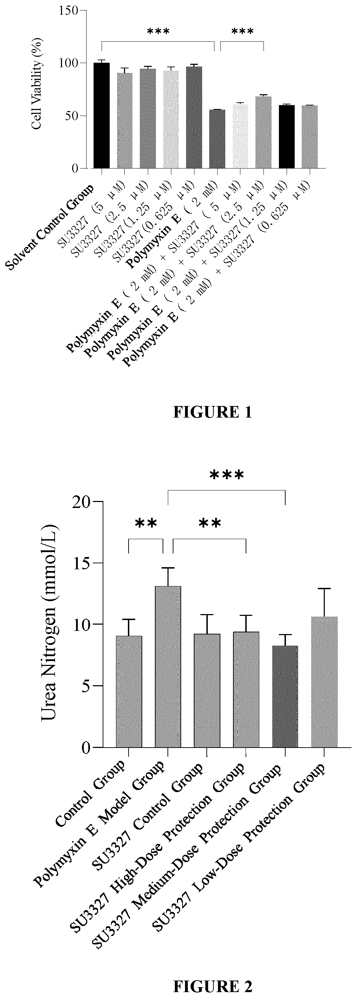

The results of reducing cytotoxicity of Polymyxin E to HEK293 cells following treatment with SU3327 were shown in Fig. 1, including the detection results of cell viability following treatment with SU3327 alone, Polymyxin E alone, or in combination. The results were analyzed in detail as follows:
Compared with control group, the cell viability of the cells treated with SU3327 alone in 5µM, 2.5µM, 1.25µM and 0.625µM was significantly changed; compared with control group, the viability of HEK293 cells following 24 hours of treatment with polymyxin in 2mM was decreased to 55.3%, showing an extremely significant difference. Compared to treatment group with polymyxin alone, the cell viability was increased to some extent following treatment with SU3327 in combination with Polymyxin E, with the cell viability increased to 68.5% following treatment with SU3327 in 2.5µM in combination with Polymyxin E, showing an extremely significant difference. It is suggested that combined treatment with SU3327 and Polymyxin E can significantly inhibit the cytotoxicity of Polymyxin E.

### Example 2 Protection by SU3327 from Nephrotoxic Injury by Polymyxin in Mice

### 1 Materials

### 1.1 Experiment Animals

C57BL/6 mice, male, 8-week-old, weighing 20-22g, purchased from Vital River Laboratory Animal Technology Co., Ltd.

### 2 Test Methods

### 2.1 Experiment Animals' Housing and Handling

The mice were housed in the National Center for Veterinary Drug Safety Evaluation of China Agricultural University, fed with routine pelleted feed and free access to water, at a controlled temperature of (22±2) °C, and a humidity of (50±10) %. Adaptive housing was performed for 1 week before experiments.

30 animals were randomly divided into 6 groups (n = 8 in each group), as follows:
Control group: the mice were intraperitoneally injected with an equivalent amount of normal saline.

Polymyxin E model group: the mice were intraperitoneally injected with Polymyxin E at a single dose of 10mg/kg body weight, twice a day, with an interval of 8 hours between doses; i.e., the mice were administered with Polymyxin E in 20mg/kg body weight per day.

SU3327 control group: the mice were intraperitoneally injected with dissolved SU3327 at a single dose of 10mg/kg body weight, once a day for 10 straight days; i.e., the mice were administered with SU3327 in 10mg/kg body weight per day.

SU3327 high-dose protection group: the mice were intraperitoneally injected with SU3327 at a single dose of 10mg/kg body weight, concurrently with Polymyxin E in 10mg/kg, twice, with an interval of 8 hours between doses; i.e., the mice were administered with SU3327 in 10mg/kg body weight per day, concurrently with Polymyxin E in 20mg/kg body weight per day.

SU3327 medium-dose protection group: the mice were intraperitoneally injected with SU3327 at a single dose of 5mg/kg body weight, concurrently with Polymyxin E in 5mg/kg, twice, with an interval of 8 hours between doses; i.e., the mice were administered with SU3327 in 5mg/kg body weight per day, concurrently with Polymyxin E in 20mg/kg body weight per day.

SU3327 low-dose protection group: the mice were intraperitoneally injected with SU3327 at a single dose of 2.5mg/kg body weight, concurrently with Polymyxin E in 10mg/kg, twice, with an interval of 8 hours between doses; i.e., the mice were administered with SU3327 in 2.5mg/kg body weight per day, concurrently with Polymyxin E in 20mg/kg body weight per day.

In the experiments above, reference was made to the previously published literature by the inventors for the method of establishing the mouse model of nephrotoxicity by Polymyxin E (Dai C, Tang S, Wang Y, Velkov T, Xiao X. Baicalein acts as a nephroprotectant that ameliorates colistin-induced nephrotoxicity by activating the antioxidant defence mechanism of the kidneys and down-regulating the inflammatory response. J Antimicrob Chemother. 2017 Sep 1;72(9):2562-2569).

Following dosing by injection for 10 straight days, the mice were euthanized by intraperitoneal injection with an overdose of sodium pentobarbital (80mg/kg) 24 hours post the last dosing, and blood and kidney tissues were collected quickly, the serum and tissues were processed as follows.

### 2.2 Determination of Biochemical Indicators

The blood samples collected from the mice were centrifuged at 3000rpm for 10min, and the supernatant was aspirated. The changes of urea nitrogen in serum and creatinine in serum were detected by an automatic biochemical detector.

### 2.3 Histopathological Examination and Semiquantitative Scoring for Kidney

The isolated, fresh kidney tissues were washed with normal saline, sampled and fixed in 10% neutral formalin for 48h,after being flushed with water, and then dehydrated, cleared, immersed in paraffin, embedded, sectioned and stained in sequence . The pathological changes in kidney tissues were observed and photographed under a microscope. HE staining: the paraffin sections were taken, and placed in an oven at a constant temperature of 60°C for 30min; followed by paraffin removal and dehydration with ethanol, and immersing with double distilled water; hematoxylin staining for 10min, rinsing with tap water, and differentiation with acid alcohol; flushing with running water until the sections turned blue; 95% ethanol for 30s, alcoholic eosin staining for 30s, 95% ethanol for 30s, and 100% ethanol for 30s; clearing with Xylene, and sealing with neutral gum.

Following HE staining, damages to the renal tissues were evaluated using a semiquantitative scoring (SQS). The SQS scoring were conducted with reference to our previously published literature (Antimicrob Agents Chemother, 2014 Jul;58(7):4075-85), with criteria detailed below: SQS = Grade Score × Percentage Damage Score; in which grade 1 (score 1): mild tubular damage, i.e., tubular dilation, prominent nuclei, and emergence of a few tubular casts in renal tubules; grade 2 (score 4): severe tubular damage, i.e., necrosis of tubular epithelial cells and emergence of numerous tubular casts in renal tubules; grade 3 (score 10): acute cortical or medullary necrosis or infarction in kidney. The Percentage Damage Score of the kidney sections: < 1% = score 0, 1% ~ <5 % = score 1, 5% ~ < 10% = score 2, 10% ~ < 20% = score 3, 20% ~ < 30% = score 4, 30% ~ < 40% = score 5, ≥ 40% = score 6. Finally, the results of evaluation by SQS were as follows: SQS + 0 = no significant change (overall score < 1); SQS + 1 = mild damage (overall score 1 ~ < 15); SQS + 2 = mild to moderate damage (overall score 15 ~ <30); SQS + 3 = moderate damage (overall score 30 ~ < 45); SQS + 1 = mild to moderate damage (overall score 45 ~ < 60); SQS + 1 = severe damage (overall score ≥60).

### 2.4 Statistical Analysis

All the results are represented as means ± standard deviations. One-way analysis of variance was performed using GraphPad 9.0 software. *, P < 0.05, **, P < 0.01, ****, P < 0.001.

### 3 Test Results

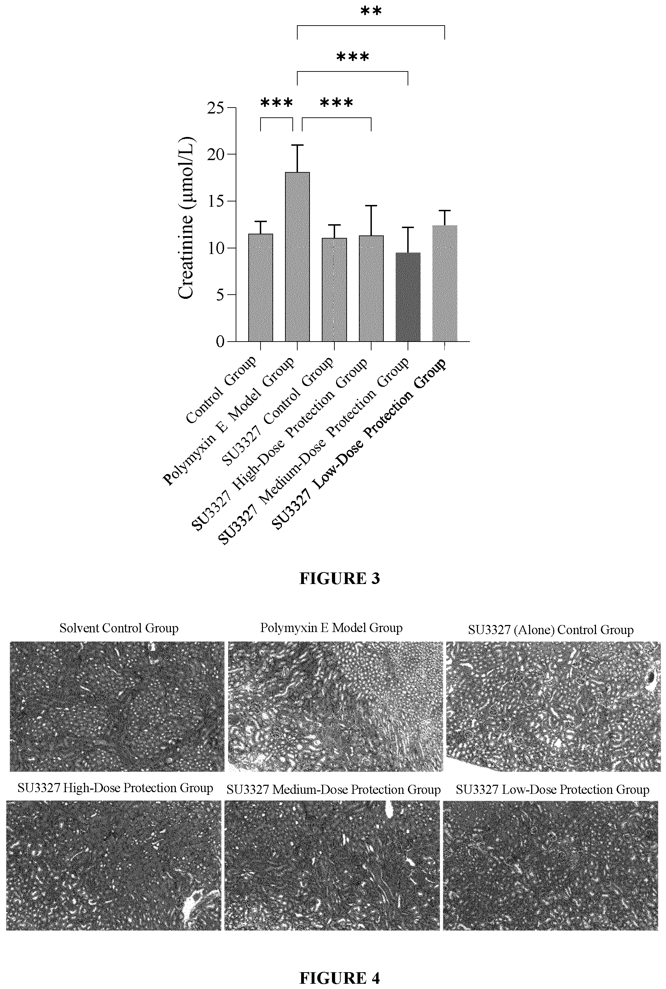

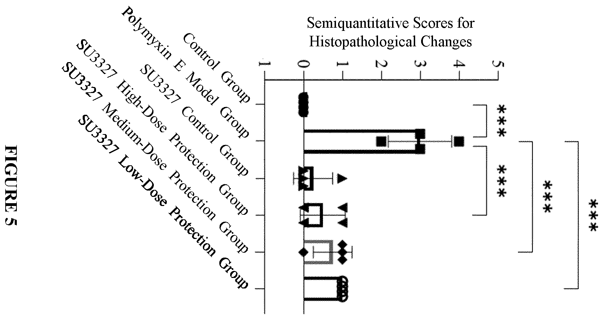

Compared to the control group, injection with Polymyxin E at 20mg/kg per day for 10 straight days resulted in significantly up-regulated levels of BUN and CRE in the serum, as well as obvious pathological damages, including renal tubular epithelial cell shedding, necrosis and tubular casts formation, with the average of pathological semi quantitative score increased to 3; compared to Polymyxin E model group, treatment with SU3327 at 2.5mg/kg, 5mg/kg, and 10mg/kg in combination with polymyxin significantly lowered the levels of urea nitrogen (as seen in Fig. 2 for the graph showing the detection results of urea nitrogen in the serum of the mice in control group, Polymyxin E model group, SU3327 control group, SU3327 high-dose protection group, SU3327 medium-dose protection group, and SU3327 low-dose protection group) and creatinine (as seen in Fig. 3 for the graph showing the detection results of creatinine in the serum of the mice in control group, Polymyxin E model group, SU3327 control group, SU3327 high-dose protection group, SU3327 medium-dose protection group, and SU3327 low-dose protection group) in the serum; Fig. 4 shows the image showing the histopathological sections of the mice in control group, Polymyxin E model group, SU3327 control group, SU3327 high-dose protection group, SU3327 medium-dose protection group, and SU3327 low-dose protection group, according to the invention; the corresponding pathological damage scores were decreased to 1 (P<0.001), 0.75 (P<0.001) and 0.5 (P<0.001), respectively (as seen in Fig. 5 for the graph showing the semi quantitative evaluation of control group, Polymyxin E model group, SU3327 control group, SU3327 high-dose protection group, SU3327 medium-dose protection group, and SU3327 low-dose protection group, according to the invention). These results indicated that combined treatment with SU3327 and Polymyxin E can significantly inhibit nephrotoxicity of Polymyxin E.

The above-mentioned examples in the present disclosure are merely exemplifications for clearly illustrating the disclosure and are not intended to limit the embodiments of the disclosure. Other variations or modifications in various forms may be made by those skilled in the art to which they pertain from the foregoing description. All embodiments are not necessarily, or possibly exhaustive here. Any of modifications, equivalent substitutions, and improvements made within the spirit and principles of the disclosure are intended to be included within the scope of the claims of the disclosure.

## Claims

1. Use of a C-JUN N-terminal kinase inhibitor, SU3327, in the preparation of a medicament for reducing cytotoxicity and/or nephrotoxicity of polymyxin.

2. The use of claim 1, **characterized in that**, the polymyxin is Polymyxin E or Polymyxin B.

3. The use of claim 1 or 2, **characterized in that**, the polymyxin is Polymyxin E.

4. The use of claim 3, **characterized in that**, the C-JUN N-terminal kinase inhibitor, SU3327, and the Polymyxin E can be prepared into a composition for reducing cytotoxicity or nephrotoxicity in human or animal bodies caused by Polymyxin E.

5. The use of claim 4, **characterized in that**, a mass ratio of SU3327 to polymyxin in the composition prepared with SU3327 and polymyxin is: (0.5-2): 1.

6. The use of claim 4 or 5, **characterized in that**, the dosage form of the composition of SU3327 and polymyxin is one of tablets, capsules, sustained release tablets, controlled release tablets, oral solutions, syrups, dosage forms for injection, dripping pills, and dosage forms of lyophilized powders for injection.

7. The use of claim 4, **characterized in that**, the final, used concentration of Polymyxin E is 2mM, and the final, used concentration of SU3327 is 5µM-0.625µM, when used to reduce cytotoxicity.

8. The use of claim 7, **characterized in that**, the final, used concentration of the SU3327 is 2.5µM.

9. The use of claim 4, **characterized in that**, the final, used dose concentration of the SU3327 is 2.5-10mg/kg body weight per day, and the final, used dose of the Polymyxin E is 20mg/kg body weight per day, when used to reduce nephrotoxicity.
